# DEMANDE DE BREVET EUROPEEN

(11) **EP 1 329 521 A1**
(43) Date de publication de la demande: **23.07.2003**
(21) Numéro de dépôt: 03290128.2
(22) Date de dépôt: 20.01.2003
(51) Int. Cl.: C12Q 1/68, C12N 15/29, C07K 14/37, C12N 9/10

(54) **Procédé de détection de champignons producteurs d'ochratoxine A ou de citrininine**

(30) Priorité: 21.01.2002 FR 0200682
(71) Demandeur: Evialis, 56250 Elven (FR)
(72) Inventeur: Lebrihi, Ahmed, 31450 Noueilles (FR); Mathieu, Florence, 31750 Escalquens (FR); Borgida, Louis Patrick, 56000 Vannes (FR); Guyonvarch, Alain Marcel, 56000 Vannes (FR)
(74) Mandataire: Warcoin, Jacques

(57) **Abrégé**

L'invention se rapporte à de nouvelles séquences issues de champignons producteurs d'ochratoxine A (OTA) ou de citrinine ainsi qu'à un procédé de détection de ces types de champignons, dans lequel on recherche la présence d'une séquence selon l'invention, notamment par PCR.

## Description

L'invention se rapporte à de nouvelles séquences issues de champignons producteurs d'ochratoxine A (OTA) ou de citrinine ainsi qu'à un procédé de détection de ces types de champignons, dans lequel on recherche la présence d'une séquence selon l'invention.

La mycoflore est estimée à plus de 200 000 espèces. Parmi elles, les moisissures (*fungi imperfecti*), qui se retrouvent partout, posent de graves problèmes au niveau de la santé et de l'alimentation de l'homme et des animaux. Elles peuvent provoquer :
- l'altération des denrées alimentaires,
- des mycoses et des allergies ,
- et des intoxications par leurs métabolites appelés mycotoxines.

Les mycotoxines sont des métabolites secondaires, c'est-à-dire qu'elles ne sont pas nécessaires au développement du champignon comme le sont les acides aminés, les acides gras, les acides nucléiques ou les protéines. Mais elles peuvent provoquer des répercussions importantes sur la sécurité alimentaire et sanitaire de l'homme et des animaux.

On a pu déterminer les nombreux genres de champignons toxinogènes ainsi que les mycotoxines qu'ils peuvent produire :

| **Champignons :** | **Mycotoxines :** | **Denrées :** |
|---|---|---|
| *Aspergillus* | Aflatoxines, Stérigmatocystine, Ochratoxine A. | Maïs, arachides, graines coton, potiron, riz, coton, potiron, riz, haricots, tissus d'animaux (jambon, lard, saucisses), lait et dérivés. |
| | | |
| *Fusarium* | Trichotécènes, Zéaralénone, Fumosines, Fusarine, Moniliformine. | Blé, maïs, orge, riz, seigle, avoine, noix. |
| | | |
| *Pénicillium* | Patuline, Citrinine, Pénitrem A, Acide cyclopiazonique, Ochratoxine A. | Fruits et jus de fruits, blé, riz, fromage, noix. |
| | | |
| *Alternaria* | Alternariol, Acide ténuazonique. | Fruits, légumes et produits dérivés de pommes et tomates. |
| *Claviceps* | Alcaloïdes de l'ergot. | Blé et dérivés, seigle. |
| (D'après A. Pfohl-Leszkowicz, *in* « *Les mycotoxines dans l'alimentation: évaluation et gestion du risque ».* Ed. Tec & Doc, Paris,1999). | | |

La préoccupation concernant des mycotoxines se fait sentir de plus en en plus chez les industriels de l'alimentation et le corps médical qui doivent éviter les risques de contamination. Pour cela, il est nécessaire de disposer de techniques puissantes qui permettent de détecter la source, le champignon producteur de la mycotoxine avant que cette dernière ne soit synthétisée dans tout milieu favorable. Il est donc intéressant de disposer des techniques dites préventives contre la production de la mycotoxine. Cette méthode devra être simple d'utilisation et d'exécution, spécifique à un micro-organisme ou à un groupe de micro-organismes ayant la capacité de produire le même groupe de mycotoxines. En plus, cette méthode doit donner des réponses assez rapides et répétitives.

IL existe des méthodes de détection et de quantification des mycotoxines. Parmi celles-ci, on trouve la chromatographie en couche mince (TLC), la chromatographie liquide haute performance (HPLC) avec détection en fluorimétrie (Shephard et al. 1995). On trouve aussi l'électrophorèse capillaire, la chromatographie en phase gazeuse (CPG) avec spectrométrie de masse ainsi que les immuno-essais. Toutes ces méthodes ont leurs avantages et inconvénients. De même on connaît des méthodes pour doser l'OTA dans le sang, les aliments et les urines. Cependant, elles restent des méthodes de détection des mycotoxines après leur production. Or, il serait intéressant de prévoir tout risque de production de mycotoxines afin d'agir pour éviter cette production. Dans ce cas il faut cibler les champignons producteurs de mycotoxines

La détection des champignons producteurs de mycotoxines se fait, d'une façon conventionnelle, par dénombrement sur boite de Pétri suivi d'une identification et d'une classification morphologique et physiologique. Toutefois, cette approche est laborieuse, longue et nécessite une expérience confirmée dans la taxonomie des champignons.

Pour ces raisons, des méthodes rapides de détection de ces micro-organismes sont nécessaires. Plusieurs méthodes rapides de détection des champignons toxinogènes ont été décrites dans la littérature. Parmi celles-ci on peut citer les méthodes immunologiques, basées sur les antigènes de surfaces, et la détermination de la quantité d'ergosterol, présent dans l'échantillon, par des méthodes analytiques. Ces approches ont toutefois le désavantage de ne pas être spécifiques. Ainsi, les anticorps dirigés contre les antigènes de surface du genre *Pénicillium* réagissent avec le genre *Aspergillus* et la détermination de l'ergostérol quantifie la présence de tous les champignons, y compris les levures, dans l'échantillon.

Par contre, la méthode basée sur l'amplification par PCR (Polymerase Chain Reaction) est très spécifique. Elle permet de distinguer les sous espèces (Hamelin et al., 1993, Curr.Genet. 25:107-13) et mêmes des souches de la même espèce (Bidouchka et al., 1994, Appl. Environ. Microbiol. 59:1752-5). De plus, si les amorces sont bien choisies, cette technique permettra, dans la majorité des cas, de différencier les souches toxinogènes des non toxinogènes dans la même sous espèce. Ceci peut être réalisé en ciblant des gènes de la voie de biosynthèse des mycotoxines chez les champignons producteurs. Cependant, les connaissances de la biologie moléculaire des champignons ne sont encore que balbutiantes et parcellaires.

Actuellement, seuls des gènes de la voie de biosynthèse des trois groupes de mycotoxines: aflatoxines (et leur intermédiaire stérigmatocystine), patuline, fumonisines, ont été caractérisés.

Ainsi, on a caractérisé les gènes pks codant pour des polycétones synthétases (PKS) dans la production :
- d'aflatoxines (*pksA* ) [*Chang et al*. *1995. Mol Gen Genet. 248:270-277; Trail et al. 1995.App. Env. Microbiol. 61 :2665-2673; Mahanti et al. 1994.Americain Phytopathological Society Meeting, Albuquerque, NM.; Mahanti et al., 1996. Appl. Env. Microbiol., 1996, 62 :191-195*],
- la patuline (*6-MSAS*) [*Beck et al. (1990). Eur. J. Biochem. 192, 487-498*], et
- les fumonisines (*fum5*) [*Proctor et al. 1999. A. Fungal Genet Biol. 27:100-11*].

On peut cibler ces gènes pour la détection des champignons producteurs de ces trois mycotoxines.

Il est nécessaire de trouver de nouvelles méthodes pour la détection des champignons producteurs d'autres mycotoxines.

La Demanderesse a découvert qu'on peut détecter avec précision et rapidité des champignons producteurs d'ochratoxine A (OTA) ou de citrinine, en recherchant la présence de nouvelles séquences nucléotidiques, codant pour des motifs protéiques précis. Ces nouvelles séquences protéiques et nouvelles séquences nucléotidiques sont des objets de l'invention, et présentent une utilité industrielle très claire, en ce qu'elles peuvent être utilisées pour détecter les champignons producteurs d'ochratoxine A ou de citrinine de façon spécifique.

Une méthode de détection de tels champignons, dans laquelle on détecte la présence de séquences selon l'invention est également un objet de l'invention.

Ainsi, l'invention se rapporte à une séquence protéique isolée comprenant la séquence REALAMDPQQRX₁LX₂EX₃X₄YE, (SEQ ID N° 41) dans laquelle X₁ est choisi parmi L/V/M/I, X₂ est choisi parmi L/M, X₃ est choisi parmi V/T et X₄ est choisi parmi V/A, et comprenant également de préférence la séquence H/K/R GP S/C M/L/A T/V I/L DDTACSSS L/M V/I A/C L/V/F H/N (SEQ ID N° 42), ou à une séquence protéique isolée comprenant la séquence SEQ ID N° 43 TX₁FVEX₂HGTGTQX₃GD, dans laquelle X₁ est choisi parmi D/A, X₂ est choisi parmi C/A et X₃ est choisi parmi F/L, et comprenant également de préférence la séquence SEQ ID N° 44 (GYARGEA).

De préférence les séquences selon l'invention sont issues d'un champignon, notamment d'un champignon produisant de la citrinine ou de l'ochratoxine A.

Dans un mode de réalisation particulier de l'invention, la séquence selon l'invention comprend une séquence choisie parmi SEQ ID N° 1 à SEQ ID N° 10.

Dans un mode de réalisation préféré de l'invention, la séquence selon l'invention est une séquence choisie parmi SEQ ID N° 1 à SEQ ID N° 10.

La recherche de séquences nucléiques codant pour des séquences protéiques selon l'invention permet de détecter de façon spécifique la présence d'un champignon produisant de la citrinine ou de l'ochratoxine A dans un échantillon, en particulier biologique ou alimentaire.

Ainsi, l'invention se rapporte également à une séquence nucléique isolée codant pour une séquence protéique selon l'invention, notamment une séquence nucléique comprenant l'une des séquences SEQ ID N° 11 à SEQ ID N° 20, en particulier une séquence nucléique choisie parmi SEQ ID N° 11 à SEQ ID N° 20.

L'invention se rapporte également à une amorce utile pour l'amplification d'une séquence nucléique selon l'invention, et comprenant entre 18 et 25 bases consécutives de ladite séquence. De façon préférée, ladite amorce est choisie parmi une séquence SEQ ID N° 21 à SEQ ID N° 40.

L'invention se rapporte également à un procédé de détection d'une souche de champignon productrice de citrinine ou d'ochratoxine A dans un échantillon, comprenant l'étape de détection d'au moins une séquence nucléique selon l'invention, en particulier au moins une séquence SEQ ID N° 11 à SEQ ID N° 20 dans ledit échantillon, de préférence au moins une séquence SEQ ID N° 11 à SEQ ID N° 17 ou SEQ ID N° 18 à SEQ ID N° 20.

La détection peut s'effectuer de diverses manières, qui sont toutes connues de l'homme du métier.

Dans un cas particulièrement préféré, le procédé de détection selon l'invention comprend les étapes de :
a) amplification de l'ADN ou l'ARN présent dans ledit échantillon, notamment en utilisant au moins une amorce selon l'invention, et en particulier au moins l'une des amorces SEQ ID N° 21 à SEQ ID N° 40,
b) détection du fragment amplifié permettant d'en déduire la présence de ladite souche productrice dans ledit échantillon.

Dans un autre mode de réalisation, le procédé de détection selon l'invention comprend les étapes de :
a) hybridation de l'ADN ou l'ARN présent dans ledit échantillon avec une sonde issue de l'une des séquences nucléiques selon l'invention, notamment SEQ ID N° 11 à SEQ ID N° 20,
b) déduction de la présence de ladite souche dans ledit échantillon par présence d'un signal d'hybridation.

Une sonde est dite issue d'une séquence nucléique lorsqu'elle permet de détecter ladite séquence nucléique ou sa séquence complémentaire par hybridation dans des conditions de forte stringence.

Une hybridation dans des conditions de forte stringence signifie que les conditions de température et de force ionique sont choisies de telle manière qu'elles permettent le maintien de l'hybridation entre deux fragments d'ADN complémentaires. Ces conditions peuvent aisément être déterminées par un homme du métier, en fonction de la taille de la sonde, et du pourcentage en bases AT et GC dans la sonde.

A titre illustratif, des conditions de forte stringence de l'étape d'hybridation aux fins de définir les fragments polynucléotidiques décrits ci-dessus, sont avantageusement les suivantes.

L'hybridation ADN-ADN ou ADN-ARN est réalisée en deux étapes : (1) préhybridation à 42°C pendant 3 heures en tampon phosphate (20 mM, pH 7,5) contenant 5 x SSC (1 x SSC correspond à une solution 0,15 M NaCl + 0,015 M citrate de sodium), 50 % de formamide, 7 % de sodium dodécyl sulfate (SDS), 10 x Denhardt's, 5 % de dextran sulfate et 1 % d'ADN de sperme de saumon ; (2) hybridation proprement dite pendant 20 heures à une température dépendant de la taille de la sonde (i.e. : 42°C, pour une sonde de taille > 100 nucléotides) suivie de 2 lavages de 20 minutes à 20°C en 2 x SSC + 2 % SDS, 1 lavage de 20 minutes à 20°C en 0,1 x SSC + 0,1 % SDS. Le dernier lavage est pratiqué en 0,1 x SSC + 0,1 % SDS pendant 30 minutes à 60°C pour une sonde de taille > 100 nucléotides. Les conditions d'hybridation de forte stringence décrites ci-dessus pour un polynucléotide de taille définie, peuvent être adaptées par l'homme du métier pour des oligonucléotides de taille plus grande ou plus petite, selon l'enseignement de Sambrook et al. (1989 Molecular cloning : a laboratory manual. 2^{nd} Ed. Cold Spring Harbor Lab., Cold Spring Harbor, New York.).

De préférence, les sondes selon l'invention comprennent plus de 50 bases consécutives d'une séquence SEQ ID N° 11 à SEQ ID N° 20, de façon plus préférée plus de 75 bases, de façon encore plus préférée plus de 100 bases, de façon la plus préférée plus de 150 bases consécutives.

L'invention se rapporté également à des outils permettant la mise en oeuvre d'un procédé de détection de champignons producteurs d'ochratoxine ou de citrinine. Ainsi, l'invention se rapporte à un support ou une puce à ADN présentant à sa surface au moins une séquence nucléotidique selon l'invention, et de préférence une séquence choisie parmi SEQ ID N° 11 à SEQ ID N° 20, de préférence au moins une séquence SEQ ID N° 11 à SEQ ID N° 17 ou SEQ ID N° 18 à SEQ ID N° 20. La puce à ADN selon l'invention peut également comprendre, à sa surface, une sonde issue de l'une des séquences nucléotidiques selon l'invention, issue notamment de SEQ ID N° 11 à SEQ ID N° 20.

La puce selon l'invention peut présenter à sa surface, au moins 2 séquences selon l'invention, de préférence au moins 3, de façon plus préférée au moins 5, de façon encore plus préférée au moins 7, de façon la plus préférée toutes les séquences selon l'invention, notamment toutes les séquences SEQ ID N° 11 à SEQ ID N° 20.

Dans un mode de réalisation particulier, la puce selon l'invention présente à sa surface les séquences SEQ ID N° 11 à SEQ ID N° 17, ou des sondes issues de ces séquences.

Dans un mode de réalisation particulier, la puce selon l'invention présente à sa surface les séquences SEQ ID N° 18 à SEQ ID N° 20, ou des sondes issues de ces séquences.

La technique des puces à ADN consiste à placer sur une surface de quelques millimètres carrés ou centimètres carrés (puces à plus ou moins haute densité) plusieurs centaines de séquences d'ADN spécifiques d'un organisme donné. Ces sondes sont hybridées avec des fragments d'ADN, généralement obtenus par RT-PCR. L'hybridation éventuelle desdits fragments est alors observée, et indique la présence ou l'absence du gène exprimé, ou de l'organisme étudié.

Ainsi, pour mettre en oeuvre le procédé selon l'invention, on peut fabriquer des « amorces » spécifiques à partir d'au moins une des séquences nucléotidiques qui seront décrites ci-après, les amorces étant des fragments d'acides nucléiques comprenant environ de 18 à 25 bases et étant un fragment consécutif de la séquence nucléotidique qui permet de reconnaître et d'amplifier spécifiquement la séquence nucléotidique totale par la méthode PCR ou RT-PCR (Reverse Transcription-PCR); les amorces étant fabriquées selon les méthodes courantes connues en biologie moléculaire), ou un fragment « complémentaire » d'un tel fragment.

On extrait ensuite l'ADN ou l'ARN d'un champignon, et l'on amplifie la séquence à l'aide des deux amorces par PCR ou RT-PCR.

Les exemples non exhaustifs et non limitatifs suivants vont illustrer l'invention.

### Procédure expérimentale

Pour les exemples de 1 à 10,
a) on cultive un champignon
b) on extrait son ADN,
c) on amplifie l'ADN par PCR en utilisant des exemples non exhaustifs et non limitatifs d'amorces fabriquées à partir des séquences nucléotidiques de l'invention.

Pour ces exemples, les méthodes communes sont les suivantes :

### 1) Cultures des champignons et extraction de leur ADN :

Les champignons sont cultivés dans le milieu PDB, Potato Dextrose Broth (Lot 139844XB, Exp MAR 04, Difco Laboratories). C'est un milieu déshydraté en poudre de composition : Extrait de pomme de terre, 200 g/L; Dextrose; 20 g/L. La préparation consiste en l'utilisation de 24g de poudre par litre d'eau distillée. Le pH final est ajusté à 5.1 +/- 0.2 (à 25°C)

La culture en milieu liquide se fait dans des Erlenmeyer de 250ml à raison de 100ml de PDB dans chacun. Les Erlenmeyer ainsi remplis sont fermés avec du coton cardé puis stérilisés pendant 15 min à 120°C.

L'ensemencement des milieux liquides est réalisé à partir des spores conservées, sur gélose inclinée, à 4°C. Un morceau de gélose est alors découpé à l'aide d'une anse de platine et mis dans l'Erlenmeyer stérilement. Les cultures sont incubées sous agitation (160 tours/min) à 25°C.

Après 1 à 2 jours de croissance, selon que le champignon pousse vite ou pas, 1 à 5ml de cette préculture vont servir à ensemencer un nouvel Erlenmeyer contenant 100ml de PDB. Cette culture est mise à pousser à 25 °C sous agitation (160 tours par minutes) pendant 16 à 20 heures afin d'avoir des cellules jeunes et en phase exponentielle de croissance avant d'être récupérées pour faire l'extraction d'ADN génomique.

Le prélèvement du matériel fongique se fait par filtration sous vide. Après 16 à 20 heures de croissance, la culture liquide est filtrée sous vide (système Millipore) sur une membrane en nitrocellulose de 0.45µm. Le mycélium est récupéré sur le filtre alors que le milieu de culture passe au travers des pores de la membrane. Le matériel fongique est alors fixé à l'azote liquide et conservé jusqu'à une utilisation ultérieure à -70°C.

La lyse cellulaire est réalisée en combinant une solution chimique et une action physique.
- Peser 300 mg de mycélium dans un tube Falcon (14 ml)
- Ajouter 5 ml de tampon de lyse cellulaire et 12,5 ml de protéinase K à 20mg/ml
   Composition du tampon de lyse :
   Tris HCI, pH 7.4, 100 mM
   EDTA, pH 8.0, 20 mM
   NaCl, 250 mM
   SDS, 2 %
- Effectuer le broyage avec l'ultra thurax
- Répartir le volume dans des Eppendorfs (de 2 ml)
- Incuber 1h à 37°C
- Incuber 30 min à 65°C (retournement des tubes toutes les 10 min)
- Ajouter 1 vol de phénol/chloroforme
- Centrifuger 1h à 4000 rmp
- Récupérer la phase supérieure (noter le volume)
- Ajouter 6 ml de RNAse A (50 mg/ml)
- Incuber 2 à 3 h à 37°C
- Ajouter 1 vol de chloroforme
- Centrifuger 10 min à 4000 rpm
- Récupérer la phase supérieure (noter le volume)
- Ajouter 1 vol de chloroforme
- Centrifuger 10 min à 4000 rpm
- Récupérer la phase supérieure (noter le volume)
- Ajouter 1 vol d'isopropanol (précipitation de l'ADN)
- Centrifuger 10 min à 13000 rmp
- Eliminer le surnageant
- Laver le culot avec 100 ml d'EtOH 70%
- Eliminer l'éthanol et sécher le culot
- Resuspendre l'ADN avec 70 ml d'eau stérile
- Effectuer le dosage puis stocker à -20°C

L'extraction de l'ADN est réalisée juste après. Le protocole est le suivant:
Les 800µl de matériel fongique soniqué sont mis dans un Eppendorf stérile.
Ajouter 5µl de RNase A (à 50mg/ml, Euromedex), mélanger par inversion. Incuber 1 heure à 37 °C dans un bain-marie.
Ajouter 10 µl de protéinase K (à 20 mg/ml, Euromedex), mélanger par inversion.
Incuber 30 min à 65 °C avec inversion toutes les 10 min.
Ajouter 800µl de phénol-chloroforme-alcool isoamylique (Sigma) (sous la hotte) .
Mélanger par inversion jusqu'a émulsion.
Centrifuger 15 min à 13 000 rotations par minutes (rpm)
Prélever 750µl de phase supérieure, les transférer dans un nouveau tube Eppendorf stérile et ajouter 750µl de phénol-chloroforme.
Mélanger par inversion jusqu'à émulsion.
Centrifuger 15 min à 13 000 rpm.
Prélever 660µl de phase supérieure, les transférer dans un nouveau tube Eppendorf stérile et ajouter 660µl de chloroforme afin de piéger les résidus de phénol.
Mélanger par inversion.
Centrifuger 5 min à 13 000 rpm
Prélever 560µl de phase supérieure, les transférer dans un nouveau tube Eppendorf stérile pour effectuer une précipitation de l'ADN. Ajouter du NaCl à [150mM final],
Ajouter 2 v/v d'éthanol 95 % (à-20°C)
Incuber 1 heure à -20 °C
Centrifuger 15 min à 13 000 rpm
Vider le surnageant et laver le culot obtenu avec 50µl d'éthanol 70 %.
Centrifuger 5 min à 13 000 rpm.
Vider le surnageant et sécher le culot à l'air, le tube renversé sur du papier
Resuspendre le culot dans 50µl de tampon TE avec un cône coupé pour ne pas casser l'ADN lors du "up and down".
Tampon TE : 10mM Tris-HCl (pH 7,4)
1mM EDTA (pH 8,0)
La solution est stérilisée par filtration (filtre 0,22µm, Millipore)
Stocker l'ADN génomique à -20 °C

### 2) Amplification par PCR :

L'amplification est réalisée par utilisation d'un kit PCR : Core System I (Promega)
Composants du kit :
*Taq DNA polymerase* 10X *buffer w*/15mM MgCl₂
*PCR Nucleotide Mix:* dNTP, 10mM
*Taq DNA polymerase*, 5u/µl
Mélange réactionnel:
*amorce 1: 0.5µl
*amorce 2: 0.5µl
matrice ADN: 0.3µl
*Taq DNA polymerase* 10X *buffer w*/15mM MgCl₂: 5µl
*PCR Nucleotide Mix:* 1µl
*Taq DNA polymerase*: 0.25µl
Eau déionisée stérile: qsp 50µL (soit 42.45µl)

Pour un ADN génomique testé, mettre tous les composés dans un microtube PCR (Fisher) de 0.2µl stérile et placé dans la glace. Ajouter une goutte d'huile minérale (Sigma). L'ensemble des tubes sont placés alors dans le thermo-cycler (Stratagène). La PCR peut démarrer selon le programme suivant :
1^{ère} étape : **dénaturation initiale** 96°C pendant 5min, 1 cycle
2^{ème} étape : pendant 40 cycles
   **dénaturation** à 96°C pendant 1 minute
   **hybridation** de 60°C pendant 1 minute
   **élongations** à 72°C pendant 2min
3^{ème} étape : **élongation terminale** 72°C pendant 10min, 1 cycle
4^{ème} étape : stockage à 6°C pendant 99min, 99cycles

Les souches de champignons testées ainsi que les mycotoxines qu'elles produisent sont:

| | Mycotoxine Produite | | | | | |
|---|---|---|---|---|---|---|
| | Ochratoxine A (ota) | Citrinine (cit) | Aflatoxine (afl) | Zéaralénone (zea) | Trichothécènes (tr) | Fumon isines (fum) |
| *Aspergillus carbonarius* M333 | X | | | | | |
| *Aspergillus ochraceus* NRRL 3174 | X | | | | | |
| *Aspergillus melleus* NRRL 3519 | X | | | | | |
| *Aspergillus sulfureus* NRRL 4077 | X | | | | | |
| *Pénicillium viridicatum* NRRL 3711 | X | | | | | |
| *Pénicillium citrinum* NRRL 1843 | | X | | | | |
| *Monascus ruber* | | X | | | | |
| *Fusarium verticilloides* NRRL 6442 | | | | | | X |
| *Fusarium graminearum* NRRL 5883 | | | | X | | |
| *Fusarium culmorum* NRRL 3288 | | | | | X | |
| *Aspergillus flavus* | | | X | | | |

### Exemple 1.

Concerne la séquence nucléotidique AOKS1.1. (SEQ ID N° 11)

Les amorces sont constituées des fractions suivantes, fabriquées à partir de la séquence AOKS1.1 :

S1 : 5'-AAGGATCGTCGACCGGCGTGTA-3' (SEQ ID N° 21)

S2 : 5'-GCCGCCTCGTCCCACATATGAC-3' (SEQ ID N° 22)

### Exemple 2.

Concerne la séquence nucléotidique AOKS1. (SEQ ID N° 12)

Les amorces sont constituées des fractions suivantes, fabriquées à partir de la séquence AOKS1 :

S3 : 5'-ACGGTGAATGCCGCGTCGTAGT-3' (SEQ ID N° 23)

S4 : 5'-GCATCGTGATACCCATGCTGCG-3' (SEQ ID N° 24)

### Exemple 3.

Concerne la séquence nucléotidique AOKS9. (SEQ ID N° 13)

Les amorces sont constituées des fractions suivantes, fabriquées à partir de la séquence AOKS9 :

S5 : 5'-GGACGAGATCCAAGGCTCGCAG-3' (SEQ ID N° 25)

S6 : 5'-TGGGCACCCATGTGGAAACAGA-3' (SEQ ID N° 26)

### Exemple 4.

Concerne la séquence nucléotidique PVKS6. (SEQ ID N° 20)

Les amorces sont constituées des fractions suivantes, fabriquées à partir de la séquence PVKS6 :

S7 : 5'-ATCATGGCCTCGTGCGAAAT-3' (SEQ ID N° 39)

S8 : 5'-CGCCCGATGGATCCTGTAAG-3' (SEQ ID N° 40)

### Exemple 5.

Concerne la séquence nucléotidique PVKS9. (SEQ ID N° 14)

Les amorces sont constituées des fractions suivantes, fabriquées à partir de la séquence PVKS9 :

S9 : 5'-AACGGCTGCTGCTCGAGACT-3' (SEQ ID N° 29)

S10 : 5'-CAGAAGGTTGGTTCCCGCTG-3' (SEQ ID N° 30)

### Exemple 6.

Concerne la séquence nucléotidique PVKS11. (SEQ ID N° 18)

Les amorces sont constituées des fractions suivantes, fabriquées à partir de la séquence PVKS 11:

S11 : 5'-AGCGATTGCAGCGGGAAGT-3' (SEQ ID N° 35)

S12 : 5'-GCCCGTGTATAGACGCTCCG-3' (SEQ ID N° 36)

### Exemple 7.

Concerne la séquence nucléotidique PVKS15. (SEQ ID N° 17)

Les amorces sont constituées des fractions suivantes, fabriquées à partir de la séquence PVKS15 :

S13 : 5'-GCAGCAGCGAATTCTTCTGGA-3' (SEQ ID N° 33)
S14 : 5'-CTGGTGCCGTTACGAAGGGA-3' (SEQ ID N° 34)

### Exemple 8.

Concerne la séquence nucléotidique ACKS9. (SEQ ID N° 16)

Les amorces sont constituées des fractions suivantes, fabriquées à partir de la séquence ACKS9 :

S15 : 5'-TGCTAGCTGCGACTGGGATG-3' (SEQ ID N° 31)

S16 : 5'-GGCGTTTCAGAACCACAGCA-3' (SEQ ID N° 32)

### Exemple 9.

Concerne la séquence nucléotidique ACKS10. (SEQ ID N° 19)

Les amorces sont constituées des fractions suivantes de la séquence ACKS10 :

S17: 5'-TCCATCTCGCGTGTCAGAGC-3' (SEQ ID N° 37)

S18: 5'-ATCAAAGCCTCTTGCGCCAC-3' (SEQ ID N° 38)

### Exemple 10.

Concerne la séquence nucléotidique CTKS3. (SEQ ID N° 14)

Les amorces sont constituées des fractions suivantes, fabriquées à partir de la séquence CTKS3 :

S19 : 5'-CCTCCGTATTCGTTGGCGTC-3' (SEQ ID N° 27)

S20 : 5'-AAAGCTTGCTCTCCGCAACG-3' (SEQ ID N° 28)

Les résultats de ces 10 expériences sont résumés dans le tableau suivant (X : montre une amplification avec un signal positif):

| | **Mycotoxine produite** | | | | | | **Réponse après PCR avec les séquences testées (S)** | | | | | | | | | |
|---|---|---|---|---|---|---|---|---|---|---|---|---|---|---|---|---|
| | **ota** | **cit** | **afl** | **zea** | **tr** | **fum** | **S1** **/** **S2** | **S3** **/** **S4** | **S5** **/** **S6** | **S7** **/** **S8** | **S9** **/** **S10** | **S11** **/** **S12** | **S13** **/** **S14** | **S15** **/** **S16** | **S17** **/** **S18** | **S19** **/** **20** |
| *Aspergillus carbonarius* M333 | oui | | | | | | **X** | **X** | **X** | | | | | **X** | **X** | |
| *Aspergillus ochraceus* NRRL 3174 | oui | | | | | | **X** | **X** | **X** | | | | | | | |
| *Aspergillus melleus* NRRL 3519 | oui | | | | | | | | **X** | | | | | | | |
| *Aspergillus sulfureus* NRRL 4077 | oui | | | | | | | | **X** | | | | | | | |
| *Penicillium viridicatum* NRRL 3711 | oui | | | | | | **X** | | **X** | **X** | **X** | **X** | **X** | | | |
| *Penicillium citrinum* NRRL 1843 | | oui | | | | | | | **X** | | | | | | | **X** |
| *Monascus ruber* | | oui | | | | | | | **X** | | | | | | | **X** |
| *Fusarium verticilloides* NRRL 6442 | | | | | | oui | | | | | | | | | | |
| *Fusarium graminearum* NRRL 5883 | | | | oui | | | | | | | | | | | | |
| *Fusarium culmorum* NRRL 3288 | | | | | oui | | | | | | | | | | | |
| *Aspergillus flavus* | | | oui | | | | | | | | | | | | | |

Ces résultats montrent que :
- les amorces fabriquées à partir des séquences nucléotidique s AOKS1.1, AOKS1, PVKS6, PVKS9, PVKS11, PVKS15, ACKS9 et ACKS10 permettent de détecter des souches de champignons productrices d'ochratoxine A (OTA),
- les amorces fabriquées à partir de la séquence AOKS9 permettent de détecter à la fois les souches de champignons productrices d'ochratoxine A et les souches de champignons productrices de citrinine,
- les amorces fabriquées à partir de la séquence nucléotidique CTKS3 permettent de détecter des souches de champignons productrices de citrinine,
- les amorces fabriquées à partir de toutes ces séquences nucléotidiques ne permettent pas de détecter les souches productrices d'aflatoxine, de zéaralénone, de tricothécènes ou de fumonisines.
Les séquences nucléotidiques selon l'invention sont donc bien spécifiques et originales.

### Exemple 11.

Cet exemple est destiné à confirmer que les amorces utilisées en PCR sont aussi utilisables en RT-PCR.

On cultive les champignons comme précédemment, on extrait leur ARN et on l'amplifie par RT-PCR (PCR après « reverse transcription »). Par rapport à la PCR, on pratique les étapes initiales suivantes :

### I) Extraction d'ARN:

Peser 300 mg de mycélium, ajouter 1 ml de Trizol, broyer et laisser 5mn à température ambiante,
Ajouter 200 µl de chloroforme, mélanger 15 s et laisser 2 à 3 mn à température ambiante,
Centrifuger 15 mn à 13000 rpm à 4°C et récupérer la phase supérieure
Ajouter 500 µl d'isopropanol, mélanger et laisser 10 mn à température ambiante
Centrifuger 20 mn à 13000 rpm à 4°C, éliminer le surnageant et ajouter 1 ml d'éthanol 75 % puis vortexer
Centrifuger 5 mn à 13000 rpm à 4°C, éliminer le surnageant, sécher le culot, ajouter 50 µl d'eau DEPC et placer 10 mn à 55-60°C

### II) Reverse transcription:

Sur 5 µg d'ARN totaux ajouter:
1 µl de polydT
11 µl qsp d'eau MilliQ stérile.
Incuber le mélange 5 mn à 70°C, puis refroidir sur glace
Ajouter par la suite:
4 µl de tampon 5X
2 µl de dNTP à 100 mM
1 µl de RNAsine
1 µl d'eau MilliQ stérile
Incuber 5 mn à 37°C
Ajouter 1 µl de reverse transcriptase
Placer 1h à 42°C puis 15 mn à 70°C
Ajouter 60 µl d'eau MilliQ stérile
Analyser sur gel d'agarose

Les exemples testés sont les trois paires d'amorces correspondant respectivement aux séquences AOKS1.1, AOKS1 et AOKS9 (S1/S2 - S3/S4 - S5/S6) déjà utilisées dans les exemples précédents de PCR. Les champignons testés ont été *Aspergillus carbonarius* M333 et *Aspergillus ochraceus* NRRL 3174, deux souches productrices seulement d'ochratoxine A. Les signaux positifs (X) de l'amplification confirment que les amorces utilisées sont aussi utiles en RT-PCR, comme le montrent les résultats résumés dans le tableau suivant :

| | Mycotoxine Produite | | | | | | Réponse après RT-PCR | |
|---|---|---|---|---|---|---|---|---|
| | OTA | Cit | Afl | Zea | Tr | Fum | **S1** **/** **S2** | **S5** **/** **S6** |
| *Aspergillus carbonarius* M333 | **X** | | | | | | **X** | **X** |
| *Aspergillus ochraceus* NRRL 3174 | **X** | | | | | | **X** | **X** |

Ainsi, les exemples de la présente invention démontrent que l'utilisation de séquences selon l'invention permet de détecter les champignons producteurs d'ochratoxine A ou de citrinine de façon spécifique, sans détecter les champignons produisant d'autres mycotoxines, comme l'aflatoxine, la Zéaralénone, les Trichothécènes ou les Fumonisines.

## Revendications

1. Séquence protéique isolée comprenant la séquence REALAMDPQQRX₁LX₂EX₃X₄YE, (SEQ ID N° 41) dans laquelle X₁ est choisi parmi L/V/M/I, X₂ est choisi parmi L/M, X₃ est choisi parmi V/T et X₄ est choisi parmi V/A.

2. Séquence selon la revendication 1, **caractérisée en ce qu'**elle comprend en outre la séquence SEQ ID N° 42 +(aa positif) G P S/C M/L/A T/V I/L D D T A C S S S L/M V/I A/C L/V/F H/N.

3. Séquence protéique isolée comprenant la séquence SEQ ID N° 43 TX₁FVEX₂HGTGTQX₃GD, dans laquelle X₁ est choisi parmi D/A, X₂ est choisi parmi C/A et X₃ est choisi parmi F/L.

4. Séquence selon la revendication 3, **caractérisée en ce qu'**elle comprend en outre la séquence SEQ ID N° 44 GYARGEA.

5. Séquence selon l'une des revendications 1 à 4 **caractérisée en ce qu'**elle est issue d'un champignon, en particulier produisant de la citrinine ou de l'ochratoxine A.

6. Séquence selon l'une des revendications 1 à 5, **caractérisée en ce qu'**elle comprend une séquence choisie parmi SEQ ID N° 1 à SEQ ID N° 10.

7. Séquence selon l'une des revendications 1 à 6, **caractérisée en ce qu'**il s'agit d'une séquence choisie parmi SEQ ID N° 1 à SEQ ID N° 10.

8. Séquence nucléique isolée codant pour une séquence protéique selon l'une des revendications précédentes.

9. Séquence nucléique selon la revendication précédente, **caractérisée en ce qu'**elle est choisie parmi SEQ ID N° 11 à SEQ ID N° 20.

10. Amorce pour l'amplification d'une séquence nucléique selon l'une des revendications 8 ou 9, comprenant entre 18 et 25 bases consécutives de ladite séquence.

11. Amorce selon la revendication 10, choisie parmi SEQ ID N° 21 à SEQ ID N° 40.

12. Procédé de détection d'une souche de champignon productrice de citrinine ou d'ochratoxine A dans un échantillon, comprenant l'étape de détection d'au moins une séquence nucléique selon l'une des revendications 8 ou 9 dans ledit échantillon.

13. Procédé de détection selon la revendication 12, comprenant les étapes de :
a) amplification de l'ADN ou l'ARN présent dans ledit échantillon, notamment en utilisant au moins une amorce selon la revendication 10 ou 11,
b) détection du fragment amplifié permettant d'en déduire la présence de ladite souche productrice dans ledit échantillon.

14. Procédé de détection selon la revendication 12, comprenant les étapes de :
a) hybridation de l'ADN ou l'ARN présent dans ledit échantillon avec au moins une sonde issue de l'une des séquences nucléiques selon la revendication 8 ou 9,
b) déduction de la présence de ladite souche dans ledit échantillon par présence d'un signal d'hybridation.

15. Puce à ADN présentant à sa surface au moins une séquence selon la revendication 8.

16. Puce selon la revendication 15, présentant à sa surface au moins une séquence selon la revendication 9, ou une sonde issue d'une séquence selon la revendication 9.

17. Puce selon la revendication 16, présentant à sa surface au moins 2 séquences selon la revendication 9, ou deux sondes issues de deux séquences selon la revendication 9.


LISTE DE SEQUENCES

<110> EVIALIS

<120> Procédé de détection de champignons producteurs
      d'ochratoxine A ou de citrininine

<130> D19783

<150> FR 02 00682
<151> 2002-01-21

<160> 44

<170> PatentIn Ver. 2.1

<210> 1
<211> 240
<212> PRT
<213> Aspergillus ochraceus

<400> 1
Arg Glu Ala Leu Ala Met Asp Pro Gln Gln Arg Leu Leu Leu Glu Val
  1               5                  10                  15

Val Tyr Glu Ser Leu Asp Arg Ala Gly Leu Gln Leu Lys Arg Leu Gln
             20                  25                  30

Gly Ser Ser Thr Gly Val Tyr Cys Gly Ala Met Asn Tyr Asp Tyr Ser
         35                  40                  45

Gln Leu Leu Leu Ser Asp Gln Asp Ser Val Pro Pro Phe Met Ile Ser
     50                  55                  60

Gly Gly Ala Pro Ser Met Leu Ser Thr Arg Ile Ser Tyr Tyr Phe Asp
 65                  70                  75                  80

Trp Lys Gly Pro Ser Leu Val Leu Asp Thr Ala Cys Ser Ser Ser Leu
                 85                  90                  95

Ile Ala Leu His Leu Ala Val Gln Ala Leu Gln Lys Gly Asp Cys Ser
            100                 105                 110

Met Ala Val Ala Ala Gly Ser Ser Leu Ile Leu Ser Val Asp Gly Tyr
        115                 120                 125

Val Ser Ser Ser Gln Ala Asn Met Met Ser Pro Ser Gly Arg Ser His
    130                 135                 140

Met Trp Asp Glu Ala Ala Asp Gly Tyr Ala Arg Gly Glu Gly Val Ala
145                 150                 155                 160

Ala Val Ile Leu Lys Arg Leu Ser Asp Ala Val Arg Asp Gly Asp Gln
                165                 170                 175

Ile Asp Ala Val Ile Arg Ala Thr Gly Val Asn Cys Asp Gly Arg Thr
            180                 185                 190

Lys Ser Leu Thr Met Pro Asn Gly Leu Ala Gln Gly Glu Leu Ile Ser
        195                 200                 205

Ser Thr Tyr Ala Ala Ala Gly Leu Asp Pro Leu Asn Pro Ala Asp Arg
    210                 215                 220

Cys Gln Phe Phe Glu Ala His Gly Thr Gly Thr Gln Phe Gly Asp Thr
225                 230                 235                 240

<210> 2
<211> 239
<212> PRT
<213> Aspergillus ochraceus

<400> 2
Arg Glu Ala Leu Ala Met Asp Pro Gln Gln Arg Val Leu Leu Glu Thr
1               5                   10                  15

Val Tyr Glu Ala Val Glu Ala Ala Gly Leu Arg Leu Glu Asp Leu Arg
            20                  25                  30

Gly Ser Pro Thr Gly Ile Phe Cys Gly Val Met Cys Asp Asp Tyr Gln
        35                  40                  45

Thr Ile Gln Gln Arg Asp Val Ser Glu Leu Pro His Tyr Thr Ala Thr
    50                  55                  60

Gly Thr Ala Arg Ser Ile Thr Ser Asn Arg Val Ser Tyr Phe Phe Asp
65                  70                  75                  80

Trp His Gly Pro Ser Met Thr Ile Asp Thr Ala Cys Ser Ser Ser Leu
                85                  90                  95

Val Ala Leu His Leu Ala Ala Lys Ala Leu His Asp Gly Glu Cys Arg
            100                 105                 110

Val Val Val Ala Ser Gly Thr Asn Leu Ile Leu Ala Pro Asn Met Tyr
        115                 120                 125

Ile Ser Glu Ser Lys Leu Ser Met Leu Ser Pro His Gly Arg Ser Arg
    130                 135                 140

Met Trp Asp Ala Gly Ala Asp Gly Tyr Ala Arg Gly Glu Gly Val Ala
145                 150                 155                 160

Ala Val Ala Leu Lys Arg Leu Ser Asp Ala Ile Ala Asp Gly Asp Ala
                165                 170                 175

Ile Glu Cys Ile Ile Pro Ala Thr His Ile Asn Gln Asp Gly Arg Ser
            180                 185                 190

Met Gly Ile Thr Met Pro Ser Ser Thr Ala Gln Thr Asp Leu Ile Arg
        195                 200                 205

Ser Thr Tyr Arg Lys Ala Gly Leu Asp Pro Ala Asn Pro Arg Asp Arg
    210                 215                 220

Cys Gln Tyr Phe Glu Ala His Gly Thr Gly Thr Gln Phe Gly Asp

225                 230                 235

<210> 3
<211> 143
<212> PRT
<213> Aspergillus ochraceus

<400> 3
Arg Glu Ala Leu Ala Met Asp Pro Gln Gln Arg Leu Leu Leu Glu Val
1               5                   10                  15

Val Tyr Glu Ala Leu Glu Asn Ala Gly Ile Thr Leu Asp Glu Ile Gln
            20                  25                  30

Gly Ser Gln Thr Ser Val Tyr Cys Gly Cys Phe Thr Asn Asp Tyr Asn
        35                  40                  45

Ala Met Thr Thr Lys Asp Leu Glu Tyr Tyr Pro Lys Tyr Thr Val Thr
    50                  55                  60

Gly Thr Gly Asn Ser Ile Leu Ala Asn Arg Ile Ser Tyr Phe Tyr Asp
65                  70                  75                  80

Leu His Gly Pro Ser Ala Thr Ile Asp Thr Ala Cys Ser Ser Ser Leu
                85                  90                  95

Val Cys Phe His Met Gly Ala Gln Ser Leu Arg Ala Gly Glu Ala Asp
            100                 105                 110

Leu Phe Asp Cys Arg Pro Gly Pro Ala Leu His Phe Asp Pro Asn Ile
        115                 120                 125

Phe Ile Thr Met Thr Asp Leu Gly Met Leu Ser Thr Asp Gly Arg
    130                 135                 140

<210> 4
<211> 239
<212> PRT
<213> Penicillium citrinum

<400> 4
Arg Glu Ala Leu Ala Met Asp Pro Gln Gln Arg Met Leu Met Glu Thr
1               5                   10                  15

Val Tyr Glu Ser Leu Cys Ala Gly Gly Gln Thr Ile Glu Gln Leu Arg
            20                  25                  30

Gly Ser Ser Thr Ser Val Phe Val Gly Val Met Cys Gly Asp Trp Glu
        35                  40                  45

Gly Met Val Gly Arg Asp Leu Glu Thr Tyr Pro Gln Tyr Gly Ala Thr
    50                  55                  60

Gly Thr Ser Arg Ser Ile Met Ser Asn Arg Ile Ser Tyr Tyr Phe Asp
65                  70                  75                  80

Trp His Gly Pro Cys Met Thr Ile Asp Thr Ala Cys Ser Ser Ser Leu

85                  90                  95

Val Ala Val His Gln Ala Ile Gln Thr Leu Arg Ser Gly Glu Ser Lys
            100                 105                 110

Val Ala Ile Ala Ala Gly Ala Asn Leu Ile Leu Ser Pro Ala Met Tyr
        115                 120                 125

Val Ala Glu Ser Lys Leu Ser Met Leu Ser Pro Ser Gly Arg Ser Arg
    130                 135                 140

Met Trp Asp Lys Asp Val Asp Gly Tyr Ala Arg Gly Glu Gly Ile Ala
145                 150                 155                 160

Ser Val Val Leu Lys Pro Leu Ser Val Ala Ile Arg Asp Asn Asp Asn
                165                 170                 175

Ile Glu Cys Leu Ile Arg Gly Thr Gly Val Asn Gln Asp Gly Arg Thr
            180                 185                 190

Pro Gly Leu Thr Met Pro Ser Gly Asn Ala Gln Ala Ser Leu Ile Arg
        195                 200                 205

Asp Thr Tyr Arg Arg Ala Asn Leu Asn Ile Asp Asp Pro Lys Asp Trp
    210                 215                 220

Pro Gln Phe Phe His Ala His Gly Thr Gly Thr Gln Leu Gly Asp
225                 230                 235

<210> 5
<211> 126
<212> PRT
<213> Penicillium viridicatum

<400> 5
Arg Glu Ala Leu Ala Met Asp Pro Gln Gln Arg Leu Leu Leu Glu Thr
1               5                   10                  15

Val Tyr Glu Gly Leu Glu Thr Ser Gly Ile Pro Met Glu Lys Leu Gln
            20                  25                  30

Gly Ser Asn Thr Gly Val Tyr Val Gly Leu Met Thr Asn Asp Tyr Ala
        35                  40                  45

Asp Met Leu Gly Arg Asp Val Gln Asn Phe Pro Thr Tyr Phe Ala Ser
    50                  55                  60

Gly Thr Ala Arg Ser Ile Leu Ser Asn Arg Leu Ser Tyr Phe Phe Asp
65                  70                  75                  80

Trp Arg Gly Pro Ser Met Thr Ile Asp Thr Ala Cys Ser Ser Ser Leu
                85                  90                  95

Ile Ala Leu His Gln Ala Val Gln Ser Leu Arg Ser Gly Glu Thr Asp
            100                 105                 110

Val Ala Val Ala Ala Gly Thr Asn Leu Leu Leu Gly Pro Glu
        115                 120                 125

<210> 6
<211> 185
<212> PRT
<213> Aspergillus carbonarius

<400> 6
Arg Glu Ala Leu Ala Met Asp Pro Gln Gln Arg Leu Leu Leu Glu Val
1               5                   10                  15

Val Tyr Glu Ser Leu Glu Thr Ala Gly Leu Gln Met Glu Gly Leu Arg
            20                  25                  30

Gly Ser Asp Thr Ala Val Phe Cys Gly Leu Met Asn Thr Asp Tyr Gly
        35                  40                  45

Asn Leu Ala Ser Cys Asp Trp Asp Ala Ile Pro Thr Tyr Ile Ser Ser
    50                  55                  60

Gly Thr Ala Ala Ser Met Leu Ala Asn Arg Val Ser Phe Phe Phe Asp
65                  70                  75                  80

Trp His Gly Pro Ser Met Thr Ile Asp Thr Ala Cys Ser Ser Ser Leu
                85                  90                  95

Val Ala Leu His Gln Gly Val Gly Ala Leu Arg Ser Gly Asp Cys His
            100                 105                 110

Leu Ala Val Ile Ala Ala Ser Asn Leu Ile Phe Ser Pro Arg Glu Tyr
        115                 120                 125

Ile Ala Ala Ser Lys Met His Leu Leu Ser Pro Thr Gly Arg Cys Arg
    130                 135                 140

Met Trp Asp Glu Asn Ala Asp Gly Tyr Ala Arg Gly Glu Gly Ile Ala
145                 150                 155                 160

Ala Val Val Leu Lys Arg Leu Gly Glu Ala Ile Ala Asp Gly Asp Pro
                165                 170                 175

Ile Glu Ser Val Ile Arg Ala Thr Gly
            180                 185

<210> 7
<211> 151
<212> PRT
<213> Penicillium viridicatum

<400> 7
Arg Glu Ala Leu Ala Met Asp Pro Gln Gln Arg Ile Leu Leu Glu Val
1               5                   10                  15

Ala Tyr Glu Ala Leu Glu Thr Ser Gly His Thr Leu Ser Asp Leu Ser
            20                  25                  30

Gly Ser Asp Thr Gly Val Phe Val Gly Leu Met Ser Gln Asp Tyr Phe
        35                  40                  45

Ala Leu Asn Gly Gln Asp Val Asn Ser Val Pro Thr Tyr Ala Ala Ser
    50                  55                  60

Gly Thr Ala Ala Ser Asn Ala Ser Ser Arg Leu Ser Tyr Phe Phe Asn
65                  70                  75                  80

Trp His Gly Pro Ser Met Thr Ile Asp Thr Ala Cys Ser Ser Ser Met
                85                  90                  95

Val Ala Val Asn Glu Ala Val Gln Ser Leu Arg Asn Gly Thr Ser Arg
            100                 105                 110

Val Ala Val Ala Cys Gly Thr Asn Leu Cys Leu Ser Xaa Phe Thr Phe
        115                 120                 125

Ile Thr Leu Ser Lys Leu Ser Met Leu Leu Pro Thr Ser Arg Cys His
    130                 135                 140

Met Trp Asp Cys His Ala Asp
145                 150

<210> 8
<211> 124
<212> PRT
<213> Penicillium viridicatum

<400> 8
Gln Arg Leu Gln Arg Glu Val Asn Leu Ile Leu Gly His Glu Phe Met
1               5                   10                  15

Ser Xaa Met Ser Met Met Asn Val Arg Phe Leu Ser Ser Asp Gly Arg
            20                  25                  30

Cys His Thr Phe Asp Glu Lys Ala Asn Gly Tyr Ala Arg Gly Glu Ala
        35                  40                  45

Ala Gly Cys Leu Ile Leu Lys Leu Ala Lys Ala Leu His Asp Arg Asp
    50                  55                  60

Asn Ile Arg Ala Val Ile Arg Gly Thr Gly Ser Asn Gln Asp Gly Arg
65                  70                  75                  80

Thr Ala Gly Ile Thr Leu Pro Asn Gly Ala Ala Gln Glu Thr Leu Ile
                85                  90                  95

Arg Ser Val Tyr Thr Arg Ala Gly Leu Asp Pro Ser Glu Thr Asp Phe
            100                 105                 110

Val Glu Ala His Gly Thr Gly Thr Gln Phe Gly Asp
        115                 120

<210> 9
<211> 154
<212> PRT
<213> Aspergillus carbonarius

<400> 9

Lys Gly Pro Ser Val Thr Val Asp Thr Ala Cys Ser Gly Ser Leu Val
1               5                   10                  15

Ala Leu His Leu Ala Cys Gln Ser Leu Arg Thr Gly Asp Ala Ser Met
            20                  25                  30

Ala Val Ala Ala Gly Val Asn Val Ile Leu Ser His Glu Phe Met Ser
        35                  40                  45

Thr Met Thr Met Met Lys Phe Leu Ser Pro Asn Gly Arg Cys Arg Thr
    50                  55                  60

Phe Asp Glu Asn Ala Asp Gly Tyr Ala Arg Gly Glu Ala Ile Gly Cys
65                  70                  75                  80

Leu Ile Leu Lys Pro Leu Lys Asn Ala Val Arg Asp Gly Asp His Ile
                85                  90                  95

His Ala Ile Ile Arg Gly Ser Gly Ser Asn Gln Asp Gly Arg Thr Pro
            100                 105                 110

Gly Ile Thr Leu Pro Ser Gly Val Ala Gln Glu Ala Leu Ile Arg Arg
        115                 120                 125

Val Tyr Gln Met Ala Cys Leu Asn Pro Ala Asp Thr Asp Phe Val Glu
    130                 135                 140

Ala His Gly Thr Gly Thr Gln Leu Gly Asp
145                 150

<210> 10
<211> 153
<212> PRT
<213> Penicillium viridicatum

<400> 10
Met Val Val Arg Thr Gly Cys Ser Ala Gly Leu Val Ala Leu His Glu
1               5                   10                  15

Ala Cys Leu Ala Ile Ser Arg Gly Asp Cys Glu Gly Ala Ile Val Xaa
            20                  25                  30

Gly Ala Asn Leu Ile Met Ala Pro Gly Met Thr Thr Ala Met Ser Glu
        35                  40                  45

Gln Gly Val Ile Ser Pro Asp Gly Ser Cys Lys Ser Phe Ser Ala Asp
    50                  55                  60

Ala Asn Gly Tyr Ala Arg Gly Glu Ala Ile Ser Ala Ile Phe Ile Lys
65                  70                  75                  80

Pro Leu Asp Asp Ala Ile Arg Asp Gly Asn Pro Val Arg Ala Val Ile
                85                  90                  95

Arg Ser Thr Ala Ser Asn Ser Asp Gly Arg Gly Ala Gly Ile Gly Ala
            100                 105                 110

His Val Pro Asn Asp Ile Ser His Glu Ala Met Ile Arg Arg Ala Tyr
        115                 120                 125

Glu Val Ala Gly Leu Thr Asp Tyr Ser Gln Thr Ala Phe Val Glu Cys
    130                 135                 140

His Gly Thr Gly Thr Gln Phe Gly Asp
145                 150

<210> 11
<211> 720
<212> ADN
<213> Aspergillus ochraceus

<400> 11
agggaggcgt tggcgatgga tccgcaacaa cgactactgt tagaagttgt atacgaatca 60
cttgatcgag ctgggctgca gctgaaacgt ctgcaaggat cgtcgaccgg cgtgtactgc 120
ggtgctatga actacgatta cagccagctg cttctatcag accaagacag tgtgcctccg 180
tttatgatct ctggtggtgc cccgtctatg ctatcgacgc gtatttctta ctatttcgat 240
tggaaggggc cttccctcgt gttggatacg gcctgctcgt ctagtttgat tgcgctacat 300
ttagccgtac aagccctgca aaagggagat tgctcgatgg ctgttgcggc tggaagcagt 360
ctgattcttt ccgtcgatgg ctacgtttcc tcgtcccagg cgaacatgat gtcgccatcg 420
ggacgcagtc atatgtggga cgaggcggcg gatgggtatg ctcgtggtga aggcgtggcc 480
gcagtgatac tcaaacggct gtccgacgcc gtgcgagatg gtgatcagat cgatgctgtg 540
atccgggcca ccggagtcaa ctgtgacgga cgcaccaagt ctttaacgat gcccaacggg 600
ctggctcagg gtgaactcat cagctctacc tacgctgctg ctggactcga ccctttgaac 660
ccagccgacc gatgccaatt ctttgaagcc catggcaccg gcacccagtt cggcgatacc 720

<210> 12
<211> 719
<212> ADN
<213> Aspergillus ochraceus

<400> 12
agggaggcgc tggcgatgga tccgcagcag cgtgttctgc tggagacagt ctacgaagcc 60
gtcgaggccg ccggtctgcg gctggaagac ctcaggggat cgcccacagg tatcttctgt 120
ggtgtgatgt gtgacgacta tcagacgatc cagcagcgcg acgtctcaga attaccgcat 180
tacacggcta caggaacagc gcgttcgatc acctcaaacc gcgtctccta ctttttcgac 240
tggcacggcc cctctatgac cattgatacc gcctgctcat ctagcctggt tgctctacat 300
ttggcggcca aagcattgca cgacggtgaa tgccgcgtcg tagtcgcctc cgggacaaac 360
ctgatcttgg ccccaaacat gtacatttcc gaatccaaat tgagtatgct gtcgccgcac 420
ggccgcagtc gtatgtggga tgccggcgca gatggctacg cgcgtggcga aggtgtcgca 480
gccgtagccc tcaagcgact cagcgacgct atcgccgacg gcgacgctat cgaatgcatt 540
attcctgcta cccatatcaa ccaggatggt cgcagcatgg gtatcacgat gccctcttcc 600
actgcccaaa ccgacctgat ccggtcgacc taccggaagg ccggcctaga tccagccaat 660
ccgcgggatc gatgccagta ttttgaagcc catggcaccg gcacccagtt cggagatcc 719

<210> 13
<211> 484
<212> ADN
<213> Aspergillus ochraceus

<400> 13
cgggaggcgt tggcgatgga tccgcaacag cgtctgttgc tggaggttgt ctatgaagcg 60
ctagaaaatg gtaagttgca gttcggggtt agtttgaggt gaggttccta acaaagaata 120
ttagctggta tcacattgga cgagatccaa ggctcgcaga cgtctgtgta ttgcggctgc 180
ttcaccaacg actacaatgc catgacaacc aaggatctcg aatattatcc aaagtacact 240
gtcactggca ccgggaactc cattctggcc aaccgcatct cctactttta cgatcttcat 300

ggtcccagtg cgacaattga cacagcttgt tcctcgtcgc tagtctgttt ccacatgggt 360

gcccagtccc ttcgggccgg cgaggccgat cttttcgatt gtcgtccggg tccggcattg 420
cattttgacc ccaacatctt catcacgatg accgacttgg gaatgctgtc tactgacggt 480
cgct 484

<210> 14
<211> 719
<212> ADN
<213> Penicillium citrinum

<400> 14
agggaggcgt tggcgatgga cccgcagcaa cggatgttga tggagactgt atacgaatcg 60
ctatgtgctg gtggacaaac aattgagcaa ctgcgtggat catctacctc cgtattcgtt 120
ggcgtcatgt gcggtgactg ggagggaatg gtgggaagag atttggaaac atatcctcag 180
tacggagcca caggaacgag tcgaagcata atgtcgaata ggatctcgta ctatttcgat 240
tggcatggac cttgtatgac gattgacacg gcatgctcgt caagccttgt cgctgtacat 300
caggcaattc aaacccttcg cagtggagag tcaaaagtag cgattgcggc gggagcaaac 360
ttgatactga gtccagctat gtacgttgcg gagagcaagc tttctatgct atctcccagc 420
ggaagatcac gtatgtggga caaagacgtt gatggatatg cccgcggtga aggtatcgct 480
tcggtagtct taaaaccact cagtgtggca attcgagata atgataatat tgagtgcctt 540
attcgaggta cgggggttaa ccaagatggg cgaaccccag gtctcactat gcctagcggg 600
aatgcgcagg cttcgctgat tcgagatacc tatcgtcgag ctaatctcaa tattgatgat 660
cctaaagact ggccacagtt ctttcatgcc catggcaccg gcacccaact cggggatcc 719

<210> 15
<211> 379
<212> ADN
<213> Penicillium viridicatum

<400> 15
agggaagcgc tggcgatgga cccgcaacaa cggctgctgc tcgagactgt ttatgagggc 60
ctggaaacgt ccggaattcc catggaaaag ctacaagggt cgaacaccgg tgtatatgtt 120
ggcctgatga ccaacgacta tgccgatatg ttaggtcggg atgtgcagaa ctttcccact 180
tattttgcat cgggaactgc ccgaagcatt ctatccaatc gactgtccta cttcttcgac 240
tggcgcggac cctccatgac cattgatacg gcgtgttcgt ctagtctgat cgccctacac 300
caggccgtac agagcctgcg atctggtgag actgatgttg ccgtggcagc gggaaccaac 360
cttctgctgg ggccggagc 379

<210> 16
<211> 557
<212> ADN
<213> Aspergillus carbonarius

<400> 16
cgggaggcgt tggcgatgga cccgcagcaa cgtctgctat tggaggtcgt ctacgagtcc 60
ctcgaaacag caggcttgca aatggaaggt ctccgaggct ctgatacggc tgttttctgt 120
ggccttatga atacggatta tggcaacctt gctagctgcg actgggatgc gatacccacg 180
tatatctcat ctggaacggc agcatctatg ctggccaatc gtgtttcctt cttttttgac 240
tggcatggcc catctatgac tattgacact gcatgctcct ctagcttggt ggcgcttcat 300
caaggcgtag gtgccctgcg gagtggagac tgccacttag ctgtaatagc cgccagcaac 360
ctgatcttta gcccgcgaga gtacattgcc gcttctaaaa tgcacctact atcccccacc 420
gggaggtgtc ggatgtggga tgagaatgcg gatggctatg cacggggtga gggtatagct 480
gctgtggttc tgaaacgcct gggggaggcc atcgcggatg gagaccctat tgaaagtgtt 540
attcgcgcca caggggt 557

<210> 17

<211> 455
<212> ADN
<213> Penicillium viridicatum

<400> 17
agggaggcgt tggcgatgga cccgcagcag cgaattcttc tggaagtggc atacgaggcc 60
ctcgaaactt cgggccatac tctcagcgat ctgtccgggt ccgacaccgg agtatttgtc 120
ggcctcatgt cccaggacta ctttgcgctg aacggccagg acgtgaattc cgttcccacc 180
tacgctgcat ctggcactgc agctagtaac gcctcgagtc ggttatctta cttcttcaac 240
tggcatggac cgtccatgac gattgatacc gcctgttcat ctagcatggt cgctgtgaac 300
gaagccgtgc agtcccttcg taacggcacc agccgcgttg ctgttgcttg tggaacaaac 360
ctgtgtttat cggncttcac atttatcaca cttagcaagc ttagcatgtt gttaccgacc 420
tctagatgtc acatgtggga ttgccacgcg gacgg 455

<210> 18
<211> 422
<212> ADN
<213> Penicillium viridicatum

<400> 18
cagcgattgc agcgggaagt gaatttaatc ttgggtcatg agtttatgtc anccatgagc 60
atgatgaagt atgtcagaca ttacccatga ccggaaatat cagactgatt tagtgttaga 120
tttctctcct cagatggaag atgtcatacc tttgatgaaa aggctaatgg atacgcgcga 180
ggcgaggcag ctggctgtct tatccttaaa cccctagcca aggcgttgca cgaccgggat 240
aacatcaggg ctgtaatacg aggaaccggt tccaatcaag acgggcgcac tgcagggata 300
acactaccaa atggggcagc ccaagaaacc ttgattcgga gcgtctatac acgggctggt 360
ctggatccct ccgaaacaga ttttgtggag gcccacggca ccggcaccca gttcggagac 420
cc 422

<210> 19
<211> 466
<212> ADN
<213> Aspergillus carbonarius

<400> 19
tgaaaggccc cagtgtgacc gttgatacgg cttgctccgg gagtttggtg gctctccatc 60
tcgcgtgtca gagccttcgg accggggatg cctctatggc cgtcgcagcg ggagtcaatg 120
taatactcag ccacgaattc atgtcaacca tgacgatgat gaaatttctc tcacctaatg 180
gacgctgccg cactttcgat gagaacgctg atgggtatgc gcggggcgaa gccattggct 240
gtctaatact caagccattg aagaatgctg ttcgagatgg ggatcatatt cacgctatca 300
tacgaggatc agggtctaac caggacggga ggacccccgg aatcacgctc cctagtgggg 360
tggcgcaaga ggctttgata cgacgcgtat atcaaatggc atgtcttaat ccagcagata 420
ccgacttcgt tgaagcccat ggcaccggca cccaactcgg cgatcc 466

<210> 20
<211> 591
<212> ADN
<213> Penicillium viridicatum

<400> 20
ggatctccga actgggtgcc ggtgccatgg cactcgacaa acgcggtctg ggagtaatcc 60
gtgaggcctg ccacctcata cgcgcgccgg atcatggcct cgtgcgaaat atcattagga 120
acatgggcgc cgattccagc accccttccg tcactgttag atgccgtcga acggataacc 180
gctcgaactg gatttccgtc ccggatggca tcatccagag gtttaatgaa gatggcagag 240
attgcctctc ctcgggcata accattggca tcggcagaga agctcttaca ggatccatcg 300
ggcgaaatta cgccttgctc actcatggcc gttgtcatgc ctggtgccat aatcaaattg 360
gcaccgncaa ctatggctcc ctcgcagtca cctctggaaa ttgccagaca tgcttcgtgt 420
aatgcaacaa gacctgctga gcagccagta cgtactacca tgctttattt atttttctcc 480

cgcttagcaa tttgcattct atttcgggaa aaaaaaaaag aaaagaaaag aaatatggat 540
caactaacct tggtcccgtc aagtncatct atatgagact cggttgggca a 591

<210> 21
<211> 22
<212> ADN
<213> Séquence artificielle

<220>
<223> Description de la séquence artificielle: Amorce

<220>
<223> S1

<400> 21
aaggatcgtc gaccggcgtg ta 22

<210> 22
<211> 22
<212> ADN
<213> Séquence artificielle

<220>
<223> Description de la séquence artificielle: Amorce

<220>
<223> S2

<400> 22
gccgcctcgt cccacatatg ac 22

<210> 23
<211> 22
<212> ADN
<213> Séquence artificielle

<220>
<223> Description de la séquence artificielle: Amorce

<220>
<223> S3

<400> 23
acggtgaatg ccgcgtcgta gt 22

<210> 24
<211> 22
<212> ADN
<213> Séquence artificielle

<220>
<223> Description de la séquence artificielle: Amorce

<220>
<223> S4

<400> 24

gcatcgtgat acccatgctg cg                                                22

<210> 25
<211> 22
<212> ADN
<213> Séquence artificielle

<220>
<223> Description de la séquence artificielle: Amorce

<220>
<223> S5

<400> 25
ggacgagatc caaggctcgc ag                                                22

<210> 26
<211> 22
<212> ADN
<213> Séquence artificielle

<220>
<223> Description de la séquence artificielle: Amorce

<220>
<223> S6

<400> 26
tgggcaccca tgtggaaaca ga                                                22

<210> 27
<211> 20
<212> ADN
<213> Séquence artificielle

<220>
<223> Description de la séquence artificielle: Amorce

<220>
<223> S19

<400> 27
cctccgtatt cgttggcgtc                                                   20

<210> 28
<211> 20
<212> ADN
<213> Séquence artificielle

<220>
<223> Description de la séquence artificielle: Amorce

<220>
<223> S20

<400> 28
aaagcttgct ctccgcaacg                                                   20

<210> 29
<211> 20
<212> ADN
<213> Séquence artificielle

<220>
<223> Description de la séquence artificielle: Amorce

<220>
<223> S9

<400> 29
aacggctgct gctcgagact                                                    20


<210> 30
<211> 20
<212> ADN
<213> Séquence artificielle

<220>
<223> Description de la séquence artificielle: Amorce

<220>
<223> S10

<400> 30
cagaaggttg gttcccgctg                                                    20


<210> 31
<211> 20
<212> ADN
<213> Séquence artificielle

<220>
<223> Description de la séquence artificielle: Amorce

<220>
<223> S15

<400> 31
tgctagctgc gactgggatg                                                    20


<210> 32
<211> 20
<212> ADN
<213> Séquence artificielle

<220>
<223> Description de la séquence artificielle: Amorce

<220>
<223> S16

<400> 32
ggcgtttcag aaccacagca                                                    20

<210> 33
<211> 21
<212> ADN
<213> Séquence artificielle

<220>
<223> Description de la séquence artificielle: Amorce

<220>
<223> S13

<400> 33
gcagcagcga attcttctgg a                                                21


<210> 34
<211> 20
<212> ADN
<213> Séquence artificielle

<220>
<223> Description de la séquence artificielle: Amorce

<220>
<223> S14

<400> 34
ctggtgccgt tacgaaggga                                                  20


<210> 35
<211> 19
<212> ADN
<213> Séquence artificielle

<220>
<223> Description de la séquence artificielle: Amorce

<220>
<223> S11

<400> 35
agcgattgca gcgggaagt                                                   19


<210> 36
<211> 20
<212> ADN
<213> Séquence artificielle

<220>
<223> Description de la séquence artificielle: Amorce

<220>
<223> S12

<400> 36
gcccgtgtat agacgctccg                                                  20

<210> 37
<211> 20
<212> ADN
<213> Séquence artificielle

<220>
<223> Description de la séquence artificielle: Amorce

<220>
<223> S17

<400> 37
tccatctcgc gtgtcagagc                                                    20


<210> 38
<211> 20
<212> ADN
<213> Séquence artificielle

<220>
<223> Description de la séquence artificielle: Amorce

<220>
<223> S18

<400> 38
atcaaagcct cttgcgccac                                                    20


<210> 39
<211> 20
<212> ADN
<213> Séquence artificielle

<220>
<223> Description de la séquence artificielle: Amorce

<220>
<223> S7

<400> 39
atcatggcct cgtgcgaaat                                                    20


<210> 40
<211> 20
<212> ADN
<213> Séquence artificielle

<220>
<223> Description de la séquence artificielle: Amorce

<220>
<223> S8

<400> 40
cgcccgatgg atcctgtaag                                                    20


<210> 41

<211> 19
<212> PRT
<213> Séquence artificielle

<220>
<223> Description de la séquence artificielle: Séquence
      consensus

<220>

<220>
<221> VARIANT
<222> (12)
<223> X est choisi parmi L, V, M ou I

<220>
<221> VARIANT
<222> (14)
<223> X est choisi  parmi L ou M

<220>
<221> VARIANT
<222> (16)
<223> X est choisi parmi V ou T

<220>
<221> VARIANT
<222> (17)
<223> X est choisi parmi V ou A

<400> 41
Arg Glu Ala Leu Ala Met Asp Pro Gln Gln Arg Xaa Leu Xaa Glu Xaa
 1               5                  10                  15

Xaa Tyr Glu

<210> 42
<211> 20
<212> PRT
<213> Séquence artificielle

<220>
<223> Description de la séquence artificielle: Séquence
      consensus

<220>
<221> VARIANT
<222> (1)
<223> X est choisi parmi H, K ou R

<220>
<221> VARIANT
<222> (4)
<223> X est choisi parmi S ou C

<220>
<221> VARIANT
<222> (5)

<223> X est choisi parmi M, L ou A

<220>
<221> VARIANT
<222> (6)
<223> X est choisi parmi T ou V

<220>
<221> VARIANT
<222> (7)
<223> X est choisi parmi I ou L

<220>
<221> VARIANT
<222> (16)
<223> X est choisi parmi L ou M

<220>
<221> VARIANT
<222> (17)
<223> X est choisi parmi V ou I

<220>
<221> VARIANT
<222> (18)
<223> X est choisi parmi A ou C

<220>
<221> VARIANT
<222> (19)
<223> X est choisi parmi L, V ou F

<220>
<221> VARIANT
<222> (20)
<223> X est choisi parmi H ou N

<400> 42
Xaa Gly Pro Xaa Xaa Xaa Xaa Asp Asp Thr Ala Cys Ser Ser Ser Xaa
 1               5                   10                  15

Xaa Xaa Xaa Xaa
            20




<210> 43
<211> 15
<212> PRT
<213> Séquence artificielle

<220>
<223> Description de la séquence artificielle: Séquence
      consensus

<220>
<221> VARIANT
<222> (2)
<223> X est choisi parmi D ou A

<220>

<221> VARIANT
<222> (6)
<223> X est choisi parmi C ou A

<220>
<221> VARIANT
<222> (13)
<223> X est choisi parmi F ou L

<400> 43
Thr Xaa Phe Val Glu Xaa His Gly Thr Gly Thr Gln Xaa Gly Asp
1               5                   10                  15

<210> 44
<211> 7
<212> PRT
<213> Séquence artificielle

<220>
<223> Description de la séquence artificielle: Séquence
      consensus

<400> 44
Gly Tyr Ala Arg Gly Glu Ala
1               5